(19) **Europäisches Patentamt / European Patent Office / Office européen des brevets**

(11) **EP 2 907 039 B1**

(12) **EUROPEAN PATENT SPECIFICATION**

(45) Date of publication and mention
of the grant of the patent:
**19.12.2018 Bulletin 2018/51**

(21) Application number: **13846109.0**

(22) Date of filing: **09.10.2013**

(51) Int Cl.:
*G06F 19/12* (2011.01)    *G16H 50/50* (2018.01)
*G06F 19/24* (2011.01)

(86) International application number:
**PCT/US2013/064160**

(87) International publication number:
**WO 2014/059036 (17.04.2014 Gazette 2014/16)**

(54) **SYSTEMS AND METHODS FOR LEARNING AND IDENTIFICATION OF REGULATORY INTERACTIONS IN BIOLOGICAL PATHWAYS**

SYSTEME UND VERFAHREN ZUM LERNEN UND IDENTIFIZIEREN VON REGULATORISCHEN INTERAKTIONEN BEI BIOLOGISCHEN SIGNALWEGEN

SYSTÈMES ET MÉTHODES POUR L'APPRENTISSAGE ET L'IDENTIFICATION D'INTERACTIONS RÉGULATRICES DANS DES VOIES BIOLOGIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 09.10.2012  US 201261711491 P
26.11.2012  US 201261729958 P
18.01.2013  US 201361754175 P

(43) Date of publication of application:
**19.08.2015 Bulletin 2015/34**

(60) Divisional application:
**18205363.7**

(73) Proprietor: **Five3 Genomics, LLC**
**Santa Cruz, California 95060 (US)**

(72) Inventors:
• **VASKE, Charles Joseph**
**Santa Cruz, California 95060 (US)**
• **SEDGEWICK, Andrew J.**
**Santa Cruz, California 95060 (US)**
• **BENZ, Stephen Charles**
**Santa Cruz, California 95062 (US)**

(74) Representative: **Hutter, Anton et al**
**Venner Shipley LLP**
**The Surrey Technology Centre**
**The Surrey Research Park**
**40 Occam Road**
**Guildford, Surrey GU2 7YG (GB)**

(56) References cited:
**US-A1- 2006 293 859    US-A1- 2007 130 206**
**US-A1- 2010 179 798    US-A1- 2012 041 683**

## Description

### Field of the Invention

The field of the invention is computational analysis of omics data, and particularly as it relates to learning algorithms for and use of pathway analysis.

### Background of the Invention

With the advent of high-throughput genomic screening increasingly larger sets of data capturing the molecular status of cells have been obtained, and these advances allowed for an increased identification and understanding of cellular mechanisms that are altered in cancer. For example, identification of key targets frequently altered within specific tumors led to the development of more than 40 targeted therapies over the last 20 years. Unfortunately, in most cases the response rate of many of these drugs is less than 50%, highlighting the incomplete understanding of the pathways affected by these drugs. A typical example of a resistance mechanism is activation of the RAS pathway in EGFR altered colon cancer tumors, where mutated KRAS constitutively activates the RAS cascade offering growth signals that are independent of the EGFR pathway, rendering EGFR blocking therapies such as cetuximab therapy largely ineffective. Thus, it appears that the knowledge of pathway interference with cetuximab is incomplete with respect to key routes through which oncogenic signals travel within the cellular signaling networks.

Such apparent incomplete knowledge is even more vexing as numerous computational tools for integrating -omics data at a pathway level are now available. Among various other tools, several algorithms (e.g., GSEA, SPIA, and PathOlogist) are capable of successfully identifying altered pathways of interest using pathways curated from literature. Still further tools have constructed causal graphs from curated interactions in literature and used these graphs to explain expression profiles. Algorithms such as ARACNE, MINDy and CONEXIC take in gene transcriptional information (and copy-number, in the case of CONEXIC) to so identify likely transcriptional drivers across a set of cancer samples. However, these tools do not attempt to group different drivers into functional networks identifying singular targets of interest. Some newer pathway algorithms such as NetBox and Mutual Exclusivity Modules in Cancer (MEMo) attempt to solve the problem of data integration in cancer to thereby identify networks across multiple data types that are key to the oncogenic potential of samples. While such tools allow for at least some limited integration across pathways to find a network, they generally fail to provide regulatory information and association of such information with one or more effects in the relevant pathways or network of pathways. Likewise, GIENA looks for dysregulated gene interactions within a single biological pathway but does not take into account the topology of the pathway or prior knowledge about the direction or nature of the interactions.

Outside genomic analysis, probabilistic graphical models have been used extensively in network analysis with landmark uses in the form of Bayesian Networks and Markov Random Fields. Several methods have successfully learned interactions from data through many different means, including relevance networks. More recently, PARADIGM (Pathway Recognition Algorithm using Data Integration on Genomic Models) is a genomic analysis tool described in WO2011/139345 and WO/2013/062505 and uses a probabilistic graphical model to integrate multiple genomic data types on curated pathway databases. Such model system advantageously allows individual samples to be assessed alone or within the context of a cohort of interest. However, expectation-maximization (EM) parameter learning in that tool was only performed by default on observational data parameters, as the limited size of available datasets prevented robust estimation of interaction parameters. Consequently, the tool did not allow for an analysis of interaction and interrelation of multiple factors that would influence activity in a particular pathway segment, and as such failed to provide improved resolution of signal flow through cellular signaling networks.

Thus, even though numerous systems and method of learning and identification of regulatory interactions in biological pathways are known in the art, all or almost all of them suffer from one or more disadvantages. For example, heretofore known analytic tools fail to identify strength and direction of interactions of parameters that modulate activity in a path of a pathway, and with that not only fail to allow prediction of signal flow and/or interference of pathway activities, but also fail to identify potential differential usage of the parameters or pathway elements. Viewed from a different perspective, currently known tools typically only consider individual gene activities, but fail to examine statistics related to regulatory links, and therefore only provide a static model rather than a dynamic model. Consequently, known models will also not allow examination as to how different regulators within a network might produce similar cellular phenotypes despite using entirely different pathways to accomplish them. Therefore, there is still a need for improved systems and methods for learning and identification of regulatory interactions in biological pathways.

### Summary of The Invention

The present invention relates to a computer-implemented method of classifying a tissue as belonging to a sub-type specific tissue, comprising:

(a) obtaining, via an omic input interface module (120), at least one omic dataset (135) representative of the tissue;

(b) accessing, via an omic processing module (170), a biological pathway model (150) having a plurality of pathway elements comprising at least one of a DNA sequence, an RNA sequence, a protein, and a protein function, in which at least two of the elements are coupled to each other via a path having a regulatory node that controls activity along the path as a function of a plurality of regulatory parameters;

i) when the pathway element comprises a DNA sequence, the at least one of the plurality of regulatory parameters is selected from the group consisting of a transcription factor, a transcription activator, a RNA polymerase subunit, a cis-regulatory element, a trans-regulatory element, an acetylated histone, a methylated histone, and a repressor,

ii) when the pathway element comprises a RNA sequence, the at least one of the plurality of regulatory parameters is selected from the group consisting of an initiation factor, a translation factor, a RNA binding protein, a ribosomal protein, an siRNA, and a polyA binding protein, and

iii) when the pathway element comprises a protein, the at least one of the plurality of regulatory parameters is a phosphorylation, an acylation, a proteolytic cleavage, and association with at least a second protein;

(c) inferring, via the omic processing module (170), based on the at least one omic dataset (135) and the pathway model (150), a set of interaction correlations among the plurality of regulatory parameters, wherein conditional probabilities of individual links are learned and a Naive Bayes assumption is used to calculate the probability of a child node Y given the parents X1, ... Xn, wherein the probability F is calculated on the basis of the expression:

$$F(Y|X_1, \ldots, X_N) = \frac{1}{Z} P(Y) \prod_i P(X_i|Y)$$

wherein Z is a normalizing constant that corresponds to P(X1, ... Xn), the pathway model being a probabilistic model configured to use factor graphs using an independent regulation model;

(d) updating the pathway model (150) based on the learned interaction correlations, wherein the omic input interface is a computing interface configured to receive one or more of omic datasets, and wherein the omic processing module is a portion of a computing device, wherein a G-test determines statistical significance of the dependence between parents given a child distribution, and
wherein a Pearson correlation or weighted pointwise mutual information (WPMI) determines the sign of interaction for the regulatory parameters,

(e) matching the derived set of interaction correlations to an a priori known set of interaction correlations that is associated with a known sub-type specific tissue; and

(f) using the match to classify that omic dataset representative of the tissue belongs to the known sub-type specific tissue, wherein the sub-type includes drug-resistant tissue, metastatic tissue, drug-treated tissue or a clonal variant of a tissue.

The omic datasets may comprise whole genome data (135), partial genome data, or differential sequence objects, and wherein the omic datasets (135) are obtained from a genomic database (130), a BAM server (130), or a sequencing device (130).

The invention also relates to an apparatus configured to carry out the method of the invention.

Interaction correlations among the regulatory parameters are inferred based on an omic dataset and/or the pathway model. So identified interaction correlations now allow to identify the strength and direction of interactions of parameters that modulate activity in a path of a pathway. Consequently, contemplated systems and methods enable prediction of signal flow and/or interference of pathway activities, as well as potentially differential usage of the parameters or pathway elements. Viewed from a different perspective, contemplated systems and methods provide a dynamic pathway model that can be used for identification of

(even differential) signal flow through one or more pathways, as well as prediction of signal flow under various (actual or simulated) scenarios.

In one aspect of this disclosure, a learning engine comprises an omic input interface that receives one or more omic datasets (e.g., whole genome data, partial genome data, or differential sequence objects). An omic processing module is coupled with the interface and is configured to (a) accesses a pathway model that has a plurality of pathway elements (e.g. DNA sequence, RNA sequence, protein, protein function) in which two or more of the elements are coupled to each other via a path having a regulatory node that controls activity along the path as a function of a plurality of regulatory parameters, (b) obtain, via the omic input interface, at least one of the omic datasets, (c) infer, based on the at least one omic dataset and the pathway model, a set of interaction correlations among the plurality of regulatory parameters, and (d) update the pathway model based on the interaction correlations. Most typically, the learning engine further comprises or is coupled to a genomic database, a BAM server, or sequencing device.

In some embodiments, the pathway element comprises a DNA sequence and the regulatory parameter is a transcription factor, a transcription activator, a RNA polymerase subunit, a cis-regulatory element, a trans-regulatory element, an acetylated histone, a methylated histone, and/or a repressor. In other embodiments, the pathway element comprises a RNA sequence and the regulatory parameter is an initiation factor, a translation factor, a RNA binding protein, a ribosomal protein, an siRNA, and/or a polyA binding protein, and in further embodiments, the pathway element comprises a protein and the regulatory parameter is a phosphorylation, an acylation, a proteolytic cleavage, and an association with at least a second protein.

In especially preferred aspects of this disclosure, the omics processing module is configured to infer the interaction correlation using a probabilistic model, which uses a co-dependent and/or independent regulation model. Moreover, it is generally preferred that the probabilistic model further determines a significance of dependence between the plurality of the regulatory parameters and the activity of the path and/or a significance of conditional dependence between the regulatory parameters given an activity of the path. Additionally, it is contemplated that the probabilistic model further determines the sign of interaction for the regulatory parameters.

Therefore, and viewed from a different perspective, in this disclosure the inventors also contemplate a method of generating a pathway model that includes a step of obtaining, via an omic input interface, at least one omic dataset (e.g., whole genome data, partial genome data, or differential sequence objects). Contemplated methods also include another step of accessing, via an omic processing module, a pathway model having a plurality of pathway elements in which at least two of the elements are coupled to each other via a path having a regulatory node that controls activity along the path as a function of a plurality of regulatory parameters, and a further step of inferring, via the omic processing module, based on the at least one omic dataset and the pathway model, a set of interaction correlations among the plurality of regulatory parameters. In yet another step, the pathway model is updated based on the interaction correlations. Most typically, the omic datasets are obtained from a genomic database, a BAM server, or a sequencing device.

In further aspects of this disclosure subject matter, the step of inferring is based on a probabilistic model, and most preferably the probabilistic model uses a co-dependent and/or independent regulation model. Additionally, contemplated methods include a step of determining a significance of dependence between the plurality of the regulatory parameters and the activity of the path and/or a significance of conditional dependence between the regulatory parameters given an activity of the path. It is also further preferred that contemplated that such methods include a step of determining the sign of interaction for the regulatory parameters.

In further aspects of this disclosure, a method of identifying sub-type specific interaction correlations for regulatory parameters of a regulatory node in a pathway model includes a step of obtaining, via an omic input interface, at least one omic dataset representative of a sub-type tissue, and a further step of accessing, via an omic processing module, the pathway model having a plurality of pathway elements in which at least two of the elements are coupled to each other via a path having the regulatory node that controls activity along the path as a function of the plurality of regulatory parameters. Contemplated methods further include a step of deriving the sub-type interaction correlations, via the omic processing module, from the at least one omic dataset representative of the sub-type tissue by probability analysis of interactions among the plurality of regulatory parameters, and another step of presenting the derived sub-type interaction correlations in the pathway model. In especially preferred aspects, the sub-type tissue is a drug-resistant tissue, a metastatic tissue, a drug-treated tissue, or a clonal variant of a tissue.

Where desired, contemplated methods may further include a step of validating the derived sub-type interaction correlations using at least one of an *in-vitro, in-silico,* and *in-vivo* experiment.

In still further aspects of the inventive subject matter, the inventors contemplate a method of classifying an omic dataset representative of a tissue as belonging to a sub-type specific tissue. Such methods will typically comprise a step of obtaining, via an omic input interface, the omic dataset representative of the tissue, and another step of deriving, for the omic dataset, a set of interaction correlations among a plurality of regulatory parameters of a regulatory node in a pathway model. In yet another step, the derived set of interaction correlations is matched to an a priori known set of interaction correlations that is associated with a known sub-type specific tissue, and the match is then used to classify that the omic dataset representative of the tissue belongs to the known sub-type specific tissue.

Most preferably, the step of obtaining comprises generating the omic dataset representative of the tissue from a tissue sample (e.g., a tumor sample) of a tissue with unknown regulatory characteristic, and the known sub-type specific tissue is a drug-resistant tissue, a metastatic tissue, a drug-treated tissue, or a clonal variant of a tissue.

In yet another aspect of this disclosure, the inventors contemplate a method of identifying a druggable target in a pathway model having a plurality of pathway elements in which at least two of the elements are coupled to each other via a path having a regulatory node that controls activity along the path as a function of a plurality of regulatory parameters. Such methods will include the steps of (a) obtaining, via an omic input interface, an omic dataset representative of a tissue, (b) deriving, for the omic dataset, a set of interaction correlations among the plurality of regulatory parameters of the regulatory node in the pathway model, and (c) identifying a drug as affecting the activity of the path where the drug is predicted to interfere with the interaction correlations. Most typically, the regulatory node affects at least one of transcription, translation, and post-translational modification of a protein, and the drug is a commercially available drug and has a known mode of action.

In still another aspect of this disclosure, the inventors contemplate method of identifying a target pathway in a pathway model having a plurality of pathway elements in which at least two of the elements are coupled to each other via a path having a regulatory node that controls activity along the path as a function of a plurality of regulatory parameters. Such methods will preferably comprise a step of obtaining, via an omic input interface, an omic dataset representative of a tissue, a further step of deriving, for the omic dataset, a set of interaction correlations among the plurality of regulatory parameters of the regulatory node in the pathway model, and a still further step of identifying a pathway as the target pathway based on a known effect of a drug on the interaction correlation.

Most preferably, the known effect is at least one of an inhibitory effect on a kinase, an inhibitory effect on a receptor, and an inhibitory effect on transcription. Among other suitable target pathways, especially contemplated target pathways include a calcium/calmodulin regulated pathway, a cytokine pathway, a chemokine pathway, a growth factor regulated pathway, a hormone regulated pathway, MAP kinase regulated pathway, a phosphatase regulated pathway, and a Ras regulated pathway. Such methods may further include a step of providing a treatment advice based on the identified pathway.

Therefore, contemplated methods will also include a method of in silico simulating a treatment effect of a drug that includes a step of obtaining a pathway model having a plurality of pathway elements in which at least two of the elements are coupled to each other via a path having a regulatory node that controls activity along the path as a function of a plurality of regulatory parameters. Contemplated methods will further include a step of identifying a drug that is known to affect at least one regulatory parameter, and another step of altering in silico, via an omic processing module and based on the known effect of the drug, at least one of the regulatory node, the activity, and at least of the regulatory parameters in the pathway model, and yet another step of determining a secondary effect of the alteration in the pathway model. Most typically, the secondary effect is in another regulatory node, another activity, and another regulatory parameter in the pathway model.

Various objects, features, aspects and advantages of the inventive subject matter will become more apparent from the following detailed description of preferred embodiments, along with the accompanying drawing figures in which like numerals represent like components.

## Brief Description of The Drawing

Figure 1 is an exemplary schematic illustration of a learning engine according to the inventive subject matter.

Figure 2A is an exemplary schematic illustration of a factor graph structure according to the inventive subject matter, and Figure 2B schematically depicts alternative regulation models for the transcription, translation, and activation nodes.

Figure 3A is an exemplary graph of a principal component analysis (PCA) of the WPMI vectors for each interaction learned across the entire TCGA cohort. Figure 3B illustrates cluster membership of significant links labeled as activation and inhibition in the pathway, and Figure 3C shows heatmaps of the WPMI values of the centroids of the clusters show a range from strong inhibition to strong activation.

Figures 4A and 4B are cluster membership bar plots for WPMI values of significant links under informative (4A) and flat (4B) initialization.

Figure 5A is an exemplary graph depicting the percentage of unique child nodes that fail the following tests at each EM step of a run learning a full conditional probability, and Figure 5B is a schematic illustration of examples of coherent versus incoherent triplets.

Figures 6A-6C are exemplary graphs depicting Kaplan-Meier survival curves for pathway analysis using different analytic methods.

Figure 7 is an exemplary heatmap representation of G-score ranks.

Figures 8A-8B are exemplary box plots depicting WPMI signals grouped by tissue for the activating links from PPARA-RXRA and TAp73a.

## Detailed Description

The inventors have now discovered that a probabilistic graphical pathway model can be implemented in which an interrelation of regulatory parameters is statistically determined. Consequently, analysis and simulations of contemplated systems and methods will provide significantly improved accuracy, and allows for identification of differential use of regulatory elements within different pathways and/or sub-tissues.

Therefore, it should be noted that by identifying regulatory links with significantly different usage distributions within a phenotype of interest in a cohort, it is now possible to examine how different regulators within a network could produce similar cellular phenotypes despite using entirely different pathways to accomplish them. Additionally, the so learned parameters can be used as the basis for statistical tests to establish how well individual samples or subsets of the cohort follow the distribution of previously learned parameter patterns for each regulatory node.

Throughout the following discussion, numerous references will be made regarding servers, services, interfaces, portals, platforms, or other systems formed from computing devices. It should be appreciated that the use of such terms is deemed to represent one or more computing devices having at least one processor configured to execute software instructions stored on a computer readable tangible, non-transitory medium. For example, a server can include one or more computers operating as a web server, database server, or other type of computer server in a manner to fulfill described roles, responsibilities, or functions.

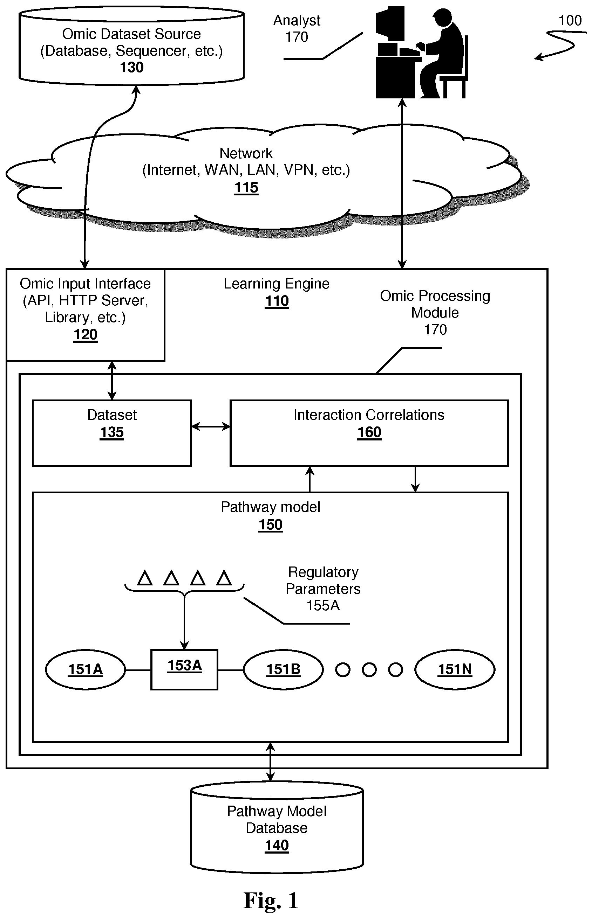

For example, **Fig. 1** exemplarily depicts ecosystem 100 that includes learning engine 110. Learning engine 110 is configured to process one or more of omic dataset 135 in view of one or more of pathway model 150. Learning engine 110 comprises two main components: omic interface 120 through which learning engine 110 obtains datasets of interest, and omic processing module 170 configured to analyze the datasets. In the example shown, learning engine 110 is illustrated as computing device accessible over network 115 (e.g., the Internet, WAN, LAN, VPN, National Lamba Rail (see URL www.nlr.net), etc.), possibly as an HTTP server farm. In some examples, learning engine 110 offers its services over network 115 for a fee. For example, learning engine 110 can expose one or more of omic input interfaces 120 to analyst 170 or other user via a cloud-based Platform-as-a-Service (PaaS), Infrastructure-as-a-Service (IaaS), Software-as-a-Service (SaaS), or other type of service. In other embodiments, learning engine 110 could be a local computing device relative to analyst 170 and configured to run one or more software instruction packages that fulfill the roles and responsibilities of learning engine 110 as discussed below.

Omic input interface 120 represents a computing interface configured to receive one or more of omic datasets 135. One example of interface 120 could include an HTTP server capable of receiving datasets 135 over network 115. For example, dataset 135 could comprise a file in a serialized format (e.g., XML), BAMBAM format, or other suitable digital formats that can be transmitted through the HTTP server. In other embodiments, interface 120 could take on the form of an Application Program Interface (API) through which data structures or their references can be passed to learning engine 110 over network 115 as an remote procedure call or even via a local library function call. It should be appreciated that omic input interface 120 can be configured to couple with one or more of omic dataset source 130, possibly operating as a database. In some embodiments, learning engine 110 comprises a genomic database or sequencing device coupled to omic input interface 120.

Omic dataset 135 can include a broad spectrum of omic data. In more preferred embodiments, omic dataset 135 represents genomic data, possibly whole genome data, partial genome data, differential sequence objects, or other genomic data. Further, omic dataset 135 can also represent other types of data including proteomic, metabolomics, lipidomics, kinomics, or other omic data modalities.

Processing module 170 represents at least a portion of a computing device coupled with omic input interface 120 and configured to analyze dataset 135 with respect to pathway model 150. One aspect of processing module 170 includes the ability to access one or more of pathway model 150, possibly from pathway model database 140 or other model source. In some embodiments, omic processing module 170 could also leverage omic input interface 120 to access pathway model database 140.

Pathway model 150 represents a digital model of activity of the target omic system to be modeled, possibly in the form N of a factor graph. Each pathway model 150 comprises a plurality of pathway elements 151A through 151N, collectively referred to as pathway elements 151. Pathway elements 151 represent stages along a path where activity takes place. Between at least two pathway elements 151, pathway elements 151A and 151B as shown for example, is a regulatory node represented by regulatory node 153A, generically referred to as regulatory node 153. Although not illustrated, there can be additional regulatory nodes 153 between each set of pathway elements 151. Thus, at least two of pathway elements 151, for example pathway elements 151A and 151B, are coupled to each other via a path having a regulatory node 153, regulatory node 153A as shown. Regulatory node 153 of pathway model 150 controls activity along the path between the elements as a function of one or more regulatory parameters 155A, generically referred to as regulatory parameters 155. One should appreciate that pathway model 150 can include any practical number of pathway elements 151, regulatory nodes 153, and regulatory parameters 155. As an example, consider scenarios where pathway elements 151 include a DNA sequence, an RNA sequence, a protein, a protein function, or other activity elements.

In scenarios where one of pathway elements 151 comprises a DNA sequence, regulatory parameters 155 can include a transcription factor, a transcription activator, a RNA polymerase subunit, a cis-regulatory element, a trans-regulatory element, an acetylated histone, a methylated histone, a repressor, or other activity parameters. Additionally, in scenarios where one of pathway elements 151 comprises an RNA sequence, regulatory parameters 155 can include an initiation factor, a translation factor, a RNA binding protein, a ribosomal protein, an siRNA, a polyA binding protein, or other RNA activity parameter. Still further, in scenarios where one of pathway elements 151 comprises a protein, then regulatory parameters 155 could include phosphorylation, an acylation, a proteolytic cleavage, or an association with at least a second protein.

Omic processing module 170 leverages pathway model 150 along with dataset 135 to infer a set of interaction correlations 160 among the plurality of regulatory parameters. One example type model that can be leveraged for inferring interaction correlations 160 includes a probabilistic model where the model configures omic processing model 170 to compare pairs of regulator parameters across multiple raw datasets 135. In some examples, regulator nodes 153 operate based on a co-dependent regulation model where learning engine 110 learns a full conditional probability table of the child given the parents. In other cases regulatory nodes 153 can operate based on an independent regulation model where the learning engine 110 learns the conditional probabilities using a Naive Bayes assumption to calculate the probability of the child node given the parent.

Contemplated probabilistic models are further configured to determine a significance of dependence between the plurality of regulator parameters 155 and the activity of the corresponding path, or signification of the conditional dependence between the regulatory parameters given an activity of the path. For example, once the conditional probabilities are calculated or otherwise established, omic processing module 150 can utilize a G-test to determine the significance. Further, the probabilistic model can be further configured to determine the sign of interaction for the regulatory parameters. Once interaction correlations 160 are established, pathway model 150 can be updated to reflect the learned interaction relationships. Consequently, it should be appreciated a learning engine will typically comprise an omic input interface that receives one or more omic datasets. Such omic input interface may be coupled to a variety of devices or systems that will in most typical cases provide omic information to an omic processing module. For example, the omic information may be derived from published data, genomic, RNomic, and/or proteomic databases, from output files of omic information databases (e.g., TCGA), as well as other devices, services, and networks that provide omic data, including DNA, RNA, and/or protein sequence databases, sequencing devices, BAM servers, etc. Consequently, it should be appreciated that the format of the data may change considerably and may be presented as whole genome data, partial genome data, or differential sequence objects.

Most typically, the omic processing module is informationally coupled with the interface and is configured to (a) access a pathway model that has a plurality of pathway elements (e.g. DNA sequence, RNA sequence, protein, protein function) in which two or more of the elements are coupled to each other via a path having a regulatory node that controls activity along the path as a function of a plurality of regulatory parameters, (b) obtain, via the omic input interface, at least one of the omic datasets, (c) infer, based on the at least one omic dataset and the pathway model, a set of interaction correlations among the plurality of regulatory parameters, and (d) update the pathway model based on the interaction correlations.

It should be recognized that the pathway models for (a) may be generated from a set of omics data, or may be obtained from previous determinations. Therefore, contemplated systems and methods will include a storage module that is coupled to the omic processing module, wherein the storage module stores one or more previously determined pathway models. It should also be recognized that the stored pathway models may correspond to 'normal' tissue or diseased tissue. Where the pathway model is from a diseased tissue, it should also be appreciated that the diseased tissue may be of a particular sub-type that is characterized by a sub-trait (e.g., sub-type that is treatment-resistant to a particular drug, sub-type that is from metastatic tissue, etc.). It is also contemplated that the omic data may be provided via the interface in numerous manners. For example, the data may be provided in a single file, or in a collection of distinct files, which may be provided by a service provider, from a library of previously stored, or from a sequencing device or sequence analysis system. Thus, the learning engine may further comprise or may be coupled to a genomic database, a BAM server, or sequencing device.

Depending on the particular path, it should be noted that the nature of the pathway element will change considerably, and with that the nature of the regulatory parameter. In general, it should be noted, however, that the regulatory parameter will determine the flow of a signal through the path from the pathway element to a downstream element. For example, where the pathway element is or comprises a DNA sequence, contemplated regulatory parameters will be those cellular entities that affect transcription (or other role) of the DNA sequence. Thus, contemplated regulatory parameters for a DNA sequence include one or more transcription factors, transcription activators, RNA polymerase subunits, cis-regulatory elements, trans-regulatory elements, (de)acetylated histones, (de)methylated histones, and/or repressors. Likewise, where the pathway element is or comprises an RNA sequence, it is contemplated that suitable regulatory parameters include factors that affect translation (or other activity) of the RNA. Consequently, such regulatory parameters include initiation factors, translation factors, RNA binding proteins, ribosomal RNA and/or proteins, siRNA, and/or polyA binding proteins. In the same way, here the pathway element is or comprises a protein, all factors affecting activity of that protein are deemed suitable regulatory parameters and may therefore include other proteins (e.g., interacting with the protein to form activated complex or complex with differential activity), chemical modification (e.g., phosphorylation, acylation, proteolytic cleavage, etc.).

With respect to inference of the set of interaction correlations among the regulatory parameters, it is generally contemplated that such inference is based on the omic dataset and/or the pathway model, and it is also generally contemplated in this disclosure that the inference is performed using a probabilistic model (e.g., co-dependent and/or independent regulation model) as set out in greater detail below. Due to the potentially very large number of possible interaction correlations, it is still further contemplated that the omic processing module will determine a level of significance of dependence between the regulatory parameters (of a single node) and the activity of the path and/or a significance of conditional dependence between the regulatory parameters (of a single node) given an activity of the path. In that way, analytic focus can be given to the interaction correlations with the statistically highest significance as is also discussed in greater detail below.

While not limiting to the inventive subject matter, the inventors also discovered that analysis of the interaction correlations and their significance can be further refined by a statistical manipulation that determines the sign (positive/activation, or negative/inhibition) of interaction for the regulatory parameters. Using the so determined interaction correlations and their influence on the path will now provide a significantly improved understanding of networks of pathways and the flow of signals through such pathways.

Therefore, and viewed from a different perspective, it should be appreciated that a pathway model can be generated by obtaining, via an omic input interface, at least one omic dataset (e.g., whole genome data, partial genome data, or differential sequence objects). An omic processing module then accesses a (e.g., previously determined) pathway model having a plurality of pathway elements in which at least two of the elements are coupled to each other via a path having a regulatory node that controls activity along the path as a function of a plurality of regulatory parameters. The omic processing module then infers, based on the omic dataset and/or the pathway model, a set of interaction correlations among the plurality of regulatory parameters, and the pathway model is subsequently updated based on the interaction correlations.

Likewise, it should be recognized that using contemplated systems and methods sub-type specific interaction correlations for regulatory parameters of a regulatory node in a pathway model can be identified. As before, at least one omic dataset representative of a sub-type tissue is obtained via an omic input interface, and an omic processing module accesses a previously determined pathway model. Sub-type interaction correlations are then derived, via the omic processing module, from the omic dataset representative of the sub-type tissue by probability analysis of interactions among the plurality of regulatory parameters as further explained in more detail below, and the derived sub-type interaction correlations are then presented (or incorporated) in the pathway model. While all kinds of sub-types of tissues are deemed suitable for use herein, especially contemplated sub-types include drug-resistant tissue, metastatic tissue, drug-treated tissue, and/or a clonal variant of a tissue. Experimental and/or theoretical experiments (e.g., *in-vitro, in-silico, in-vivo*) may then be performed to validate the derived sub-type interaction correlations. Of course, and with respect to the components and methods of such methods, the same considerations as provided above and in the following below apply.

More specifically, in the probabilistic graphical model presented herein, the states of biological molecules (e.g. proteins, mRNAs, complexes and small biomolecules) from a sample (e.g., tumor biopsy) are presented as variables. For example, for every gene, variables are used for the genome copy number of that gene, mRNA transcribed from that gene, protein derived from that gene, and in most cases a non-physical additional variable that corresponds to biological activity of a gene (as annotated in a pathway), which may be regulated by posttranslational modification of the protein. Variables can also be included that represent more abstract states, such as apoptosis, that are commonly annotated in pathways.

Causal interactions that change the state of molecules (e.g. gene transcriptional regulation, protein phosphorylation, complex formation) are represented as directed edges from the regulating variable to the regulated variable.

Therefore, for each variable Y in the probabilistic graph of the model, a factor is introduced into a joint probability model that relates the state of the variable to the state of all its regulators: $F(Y|X_1,X_2,...,X_N)$, where $X_1$ through $X_N$ are the variables that regulate $Y$. This factor is a conditional probability table: for each setting of Parents(Y), $\sum y \in F(Y = y|Parents(Y)) = 1$. Observations of individual variables, such as the genome copy number or gene expression, are modeled as separate variables, connected to the latent variable by a factor $F(Y|X)$, also a conditional probability table. The full joint probability state is then:

$$P(\Omega) = \frac{1}{Z} \prod_{Y \in \Omega} F(Y|\text{Parents}(Y))$$

where Z is a normalization constant required due to regulatory cycles in the pathway.

Given observations for a sample, one can then solve for marginal distribution of each unobserved variable, using the loopy belief propagation implementation in libDAI with inference performed in the probability space (as opposed to log space), a convergence tolerance of $10^{-9}$ and with the SEQFIX update schedule. The parameters for all F functions are learned in a machine learning process via expectation maximization in libDAI, stopping when the ratio of successive log-likelihoods is less than $10^{-10}$.

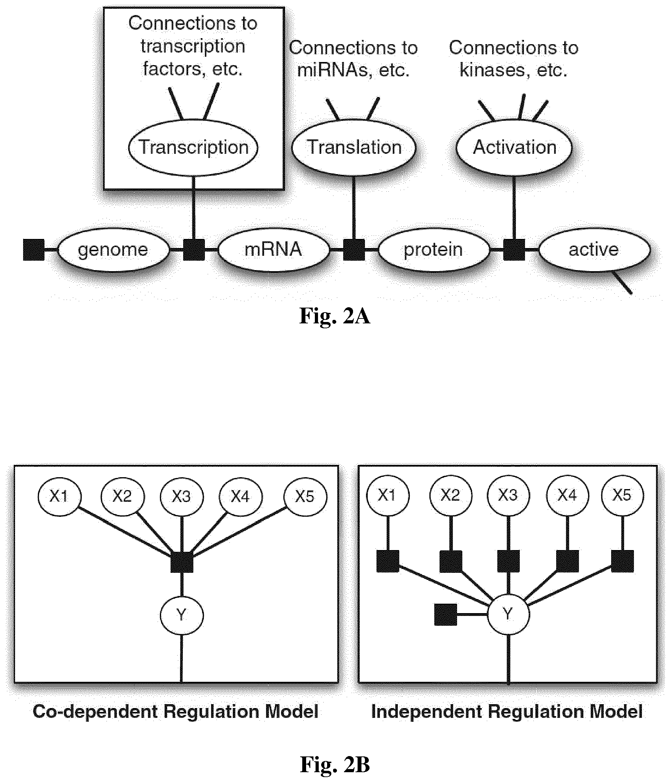

It should be appreciated that the inventors now have introduced new variables into each gene's central dogma that correspond to the transcriptional, translational and protein regulation states of each gene, as shown in **Fig. 2A** depicting a typical factor graph structure. This central dogma means that each protein-coding gene will have identical central dogma structure, and it is therefore possible to share parameters between all genes. The regulatory program is then modeled in the transcription, translation, and protein regulation variables for each gene.

## Regulation models

The previously developed algorithm (as described in WO 2013/062505 and WO 2011/139345) was extended by altering how regulation nodes are handled by the algorithm. To construct a factor graph and allow for comparison between many types of data, the previously developed algorithm discretizes the input data to down, up, or normal relative to some control. Regulation nodes collect activity signals of all of the genes involved in regulation of a given gene at some point along the path from DNA to active protein. These signals are collected in a single variable which connects to a gene's central dogma structure through a factor. Under the previously developed algorithm, regulation nodes simply take a vote of incoming signals to decide if an activation or inhibition signal was passed along.

In contrast, in the systems and methods according to this disclosure, the likelihood of each setting of the child variable Y being passed given the setting of the parent nodes $X_1,...,X_N$ is learned using a machine learning process. In the following, a co-dependent and an independent regulation model is contrasted and exemplarily shown in **Fig. 2B,** depicting alternative regulation models for the transcription, translation and activation nodes. In the Codependent Regulation Model, a full conditional probability table of the child given the parents is learned, while in the Independent Regulation Model, conditional probabilities of individual links are learned and a Naive Bayes assumption is used to calculate the probability of the child node given the parents.

More specifically, with the co-dependent regulation model of this disclosure, the probability is stored directly as a parameter in a conditional probability table for all possible settings of the parents and child. In contrast, with the independent regulation model, $P(Y)$ and $P(X_i|Y)$ are used as parameters and the product of the parameters is calculated to find the following probability:

$$F(Y|X_1, ..., X_N) = \frac{1}{Z} P(Y) \prod_i P(X_i|Y)$$

where Z is a normalizing constant that corresponds to $P(X_1,...,X_N)$. To initialize the parameters for the independent regulation model, $P(Y)$ is given an equal probability of down, up, or normal, and the initial probability for $P(X_i|Y)$ is set based on the annotation of the link in the pathway. For links labeled in the annotation as activators $P(down|down) = P(normal|normal) = P(up|up) = 0.8$, and for inhibitors $P(down|up) = P(normal|normal) = P(up|down) = 0.8$ with all the probabilities of all other settings set to 0.1. Tests were performed using a uniform distribution across all settings to evaluate the importance of using this prior knowledge from the pathway. The same simple voting procedure was then used as originally in the previously developed algorithm as the initial parameters for EM learning in the co-dependent regulation model of this disclosure. Where $\in = 0.001$, it follows that 99.9% of the probability is placed in the child state that wins the vote and 0.05% is placed in the other states as the initial likelihoods.

In addition, the inventors also allowed for 'activation' regulation of complexes and gene families between the protein and active states. Specifically, each family and complex is now modeled by a trio of variables: family/complex, regulation and active, connected with a single factor *F(active|regulation, family|complex).* Regulators of the family or complex are connected to the active variable, with either the co-dependent of this disclosure or the independent regulation model. Components of the family or complex are connected to the family/complex variable, using either a noisy-min or noisy-max factor, with $\in$ = 0.001. In contrast, only the noisy-min or noisy-max factor was used in the previously developed algorithm.

## Regulation Statistics

The inventors used G-tests to determine the statistical significance of the dependence between parents and children of regulatory links (first equation) as well as the statistical significance of the conditional dependence between parents given a child distribution (second equation):

$$G_{p-c} = 2 \sum_{i,j} O_{i,j} \ln \frac{O_{i,j}}{E_{i,j}}$$

$$= 2N \sum_{i,j} P(X_i, Y_j) \ln \frac{P(X_i, Y_j)}{P(X_i)P(Y_j)}$$

$$G_{p-p} = 2N \sum_{i,j} P(X_i, X_j|Y) \ln \frac{P(X_i, X_j|Y)}{P(X_i|Y)P(X_j|Y)}$$

It should be noted that the G-test follows the $X^2$ distribution, so that one can find P-values using $X^2$ distributions with 4 and 12 degrees of freedom for the parent-child test and the parent-parent test, respectively. P-values are adjusted for false discovery rate (FDR) and links with adjusted P<0.05 were considered significant. Although the G-test (which is proportional to the mutual information) is informative on how strong an interaction is, it does not provide details about the sign of the interaction (activation being a positive interaction, and inhibition being a negative interaction).

To obtain such information, the inventors calculated both the Pearson correlation between the parent and child, and the weighted pointwise mutual information, or WPMI (see formula below) at all possible settings of parent and child. Correlation was calculated using the joint distribution $P(X_i, Y) = P(X_i|Y)P(Y)$, and significance was calculated using the Fisher transformation. Correlation between two parents given the child was also calculated to determine if the three nodes formed a coherent or incoherent feed forward loop. To compare G-test results between groups, we took the differences of the ranks of the G statistic in each group. The significance of this statistic was calculated by performing a permutation test with 5000 random permutations of the group membership and then adjusting for FDR. For differences greater than any of those observed in the permutations, the lowest possible P-value was used as an upper bound.

$$WPMI_{i,j} = P(X_i, Y_j) \ln \frac{P(X_i, Y_j)}{P(X_i)P(Y_j)}$$

Thus, it should be recognized that the WPMI is simply each individual element of the G-score sum, and the vector of 9 WPMI values can be arranged as an easy to interpret heat map. The data can be analyzed using a HOPACH clustering algorithm (from Bioconductor), which attempts to find the number of clusters that best fits the data. This results in different numbers of clusters for each set of IPLs clustered. To find clusterings with a consistent number of clusters between all datasets, the inventors collapsed the smallest clusters by reassigning small cluster members to the closest large cluster, and collapsed small clusters in this manner to get a consistent number of clusters across all of the clusterings. This method also served to keep cluster sizes consistent across our comparisons.

## Example

There are numerous manners to produce a pathway model, and a representative model was generated from Reactome, the PID, and the NCI PID parsing of BioCarta, downloaded in BioPAX Level 3 format of February 27, 2012. That pathway model comprised 7111 proteins, 52 RNA genes, 15 miRNA genes, 7813 complexes, 1574 gene families, and 586 abstract biological processes. There were 8603 interactions changing the activation state of a molecule (3266 inhibitory), 2120 transcriptional activation links, and 397 transcriptional repression links, and there were 24129 components for the 7813 complexes, and 7170 members of the 1574 gene families.

The inventors used DAVID to perform gene set enrichment on the genes involved in interactions learned by the inventive systems and methods. To maximize number of genes recognized by DAVID, gene complexes and families were split into their component genes. Enrichment for genes involved in links was compared to a background of all of the genes in the curated pathway.

A full conditional probability table with N parents will store probabilities for all $3^{N+1}$ possible settings of parents and children. As some central genes in the curated pathway have >30 regulators, the number of parent nodes that could be attached to a child node was limited to 5 to prevent the size of these tables from becoming prohibitive. For genes regulated by more than five proteins, intermediate nodes were added to the graph to maintain this limit. Thus, a gene with 10 regulators will have two intermediate nodes with five regulators attached to each intermediate node.

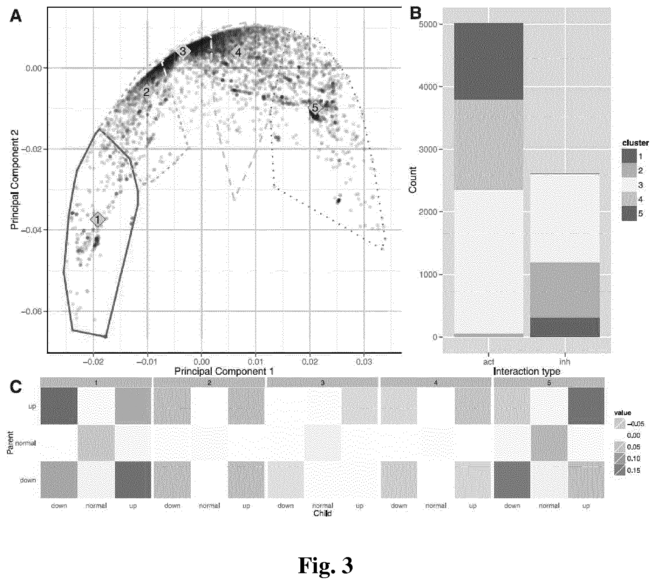

Using a dataset of 1936 TCGA tumor samples with gene expression and copy number data from 11 tissue types, interactions and regulatory interactions were learned, interaction significance was determined by a G-test, and interaction signs were determined with a correlation value as described above. Of the 9139 interactions in the pathway model that regulate a protein, 7631 (83.5%) were found to be significant at an FDR of 0.05. A principal component analysis (PCA) of the WPMI vectors for each interaction learned across the entire TCGA cohort revealed a gradient from strong inhibition to strong activation. An exemplary principal component analysis is shown in **Fig. 3A-C.** Here, panel (A) graphically depicts the principal component analysis of regulatory links in the TCGA cohort in which each point is the projection of the 9 WPMI scores for a link onto the top two principal components. The convex hulls show the membership of k-means clustering performed on the (unprojected) WPMI scores, and the cluster numbers are placed at the centroid of each cluster. Panel (B) illustrates cluster membership of significant links labeled as activation and inhibition in the pathway, and panel (C) shows heatmaps of the WPMI values of the centroids of the clusters show a range from strong inhibition (1) to strong activation (5). K-means clustering of the WPMI vectors found clusters along this gradient representing canonical interaction types ranging from strong activation to strong inhibition. Of 7631 significant links, 78 (1%) were placed in a cluster where the centroid was going the opposite direction of how the link was annotated in the pathway. The variety of WPMI vectors shows that EM was able to learn new interaction regimes that resemble activators and inhibitors as well more complex regulatory patterns.

Using statistical correlation measures (see above), the inventors then assessed each interaction as activation or inhibition and compared with the interaction type annotated in the pathway model. There were 7357 links with both significant correlation and g-scores and of those the correlation of 219 links (3%) did not agree with the direction of regulation in the pathway. This leaves 7138 (78%) links that are significant by both tests and agree with the curated links. The inventors also found that that some links had high correlation values but low significance from our g-tests, which was usually observed in cases where either the parent or child distribution greatly favored a single state.

Of the links learned by the inventive method, 1197 had significant correlation and g-scores and did not include complexes or families. For 51 of these links (4.3%), the sign of correlation coefficient disagreed with the literature. On the other hand, looking only at gene expression profiles, 1058 non-complex non-family links were found with significant correlation, but 470 (44%) disagreed with the sign of the pathway entry. For a second comparison, complexes and families were eliminated in the pathway by connecting all genes that were components of families and complexes directly to any gene regulated by those families and complexes. This flattening procedure resulted in 200921 links. We found that 165258 of these links had significantly correlated gene expression profiles, and that 81558 of the links (49.4%) had correlation that disagreed with the direction of the link in the pathway. These results indicate that the links learned by the inventive method are in significantly better agreement with the direction of the links in literature than the correlation of gene expression profiles.

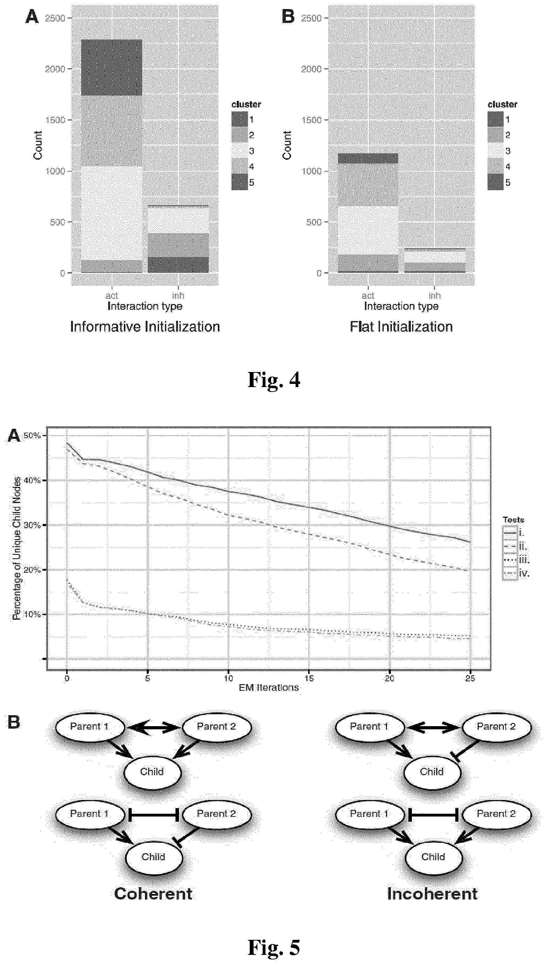

Running the PCA and clustering analysis on only WPMI scores learned from TCGA ovarian cancer (OV) patients (N=416) and without complex and family activation regulation produced very similar results to the PCA and cluster centers shown in Figs.3A and 3C, but found fewer significant links and a higher proportion of links that were annotated as activators, and learned as inhibitors or vice versa (**Fig. 4A**). When a flat initialization of $P(X_i|Y)$ = 1/3 (**Fig. 4B**) was used, the inventors found that the cluster centers again mapped to a gradient from activation to inhibition, and there were fewer significant links and a higher proportion of link direction disagreements than with initial settings that include direction information.

To test the Naive Bayes independence assumption presented in Fig. 2, systems and methods according to the inventive concept were run with both independent and codependent regulation models on the TCGA ovarian cancer samples. The inventors tested the conditional independence assumption on the expectations calculated at each EM step of the run (see **Fig. 5A**). Fig. 5A illustrates the percentage of unique child nodes that fail the following tests at each EM step of a run learning a full conditional probability (Legend: i. a test of the significance of conditional independence of any two parents given the child. ii. test i and at least one of the parents that fails is significantly linked to the child. iii. test i and the failing triplet is incoherent, iv. tests i, ii and iii. At every step of learning, fewer co-regulators were found to be dependent upon each other. Because of small feedback loops in the pathway, such as a transcription factor that regulates its own transcription, one would expect that the independence assumption will fail in some cases. Additionally, it is quite common for two very similar complexes, differing by only one molecule, to co-regulate the same child node, in which case one would also expect the conditional independence test to fail, despite there being little conflict. Consequently, the inventors divides the cases where two co-regulators fail the independence test into 'coherent' and 'incoherent' classes, as schematically shown in **Fig. 5B.** Fig. 5B schematically illustrates examples of coherent versus incoherent triplets. The arrows correspond to correlation with a pointed head for positive correlation (activation) and a flat head for negative correlation (inhibition). The interactions between parents are not found in the literature, so double sided arrows were used because the direction of that interaction was unknown.

Additionally, two co-regulators may fail the independence test even if one of the co-regulators is an insignificant regulator, owing to the strength of the other regulator. The inventors therefore also considered the subset of cases where both co-regulators are significant on their own, and the tests show that the initial parameters produced by the weighted vote method cause almost 50% of child nodes to fail the conditional independence test, but as the EM algorithm learns more likely parameter settings, fewer and fewer nodes fail the test. Combining all of our tests shows that only -5% of child nodes are likely to have codependent regulators in a meaningful way.

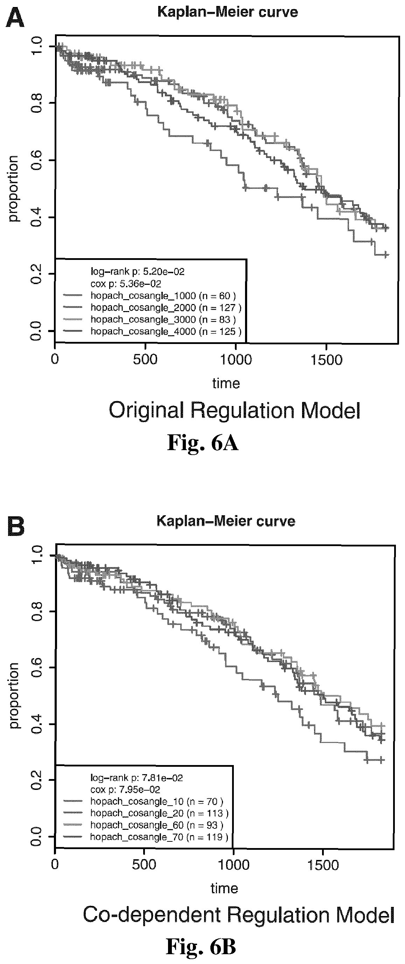

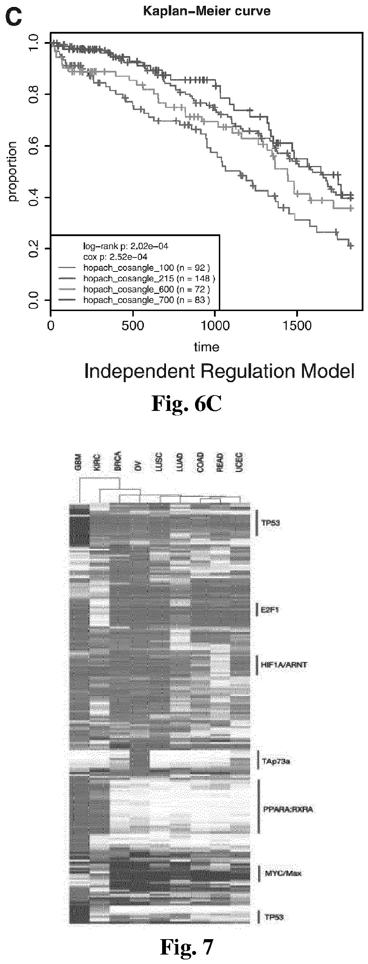

Using the ovarian cancer samples, the inventors further clustered the protein activity predictions produced by the previously developed algorithm (see WO 2013/062505 and WO 2011/139345) and those from both the co-dependent and independent regulation models. A Kaplan-Meier analysis was then performed on these clusters to see whether they had significantly differential survival profiles (**Fig. 6**). Here, Kaplan-Meier survival curves of 416 patients in the TCGA ovarian cohort clustered by Integrated Pathway Activity are shown using (Fig. 6A) the previously developed algorithm, (Fig. 6B) the inventive algorithm learning full conditional probability tables of regulatory nodes, and (Fig. 6C) the inventive algorithm learning conditional probability of single links and using a naive Bayes assumption. The inventors found that the clusters produced using independent regulation model activity predictions were the most separable by their survival (log-rank P = $2.0 \times 10^{-4}$). The inventors also performed this test using the independent regulation model with a flat initial setting for the $P(X_i|Y)$ parameters and found that it performed worse than the previously developed algorithm. Again, this indicates that the learning method requires prior knowledge about the type of interaction that is lost when using a flat initial interaction setting.

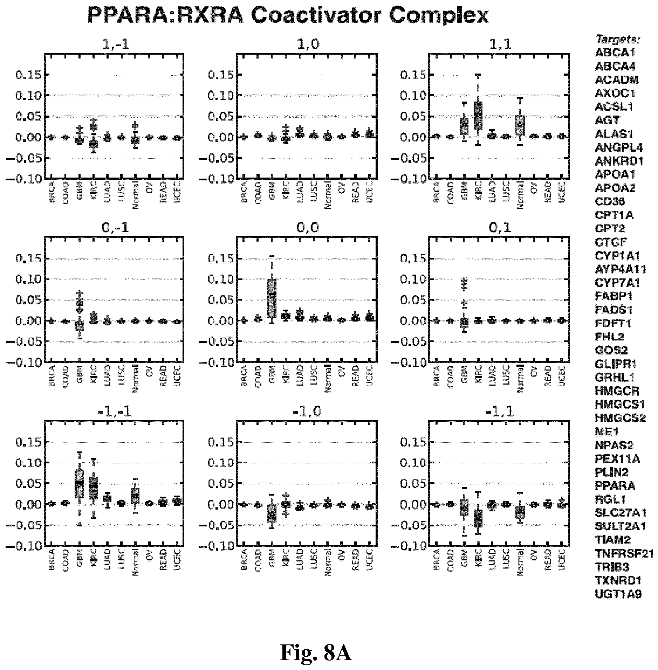

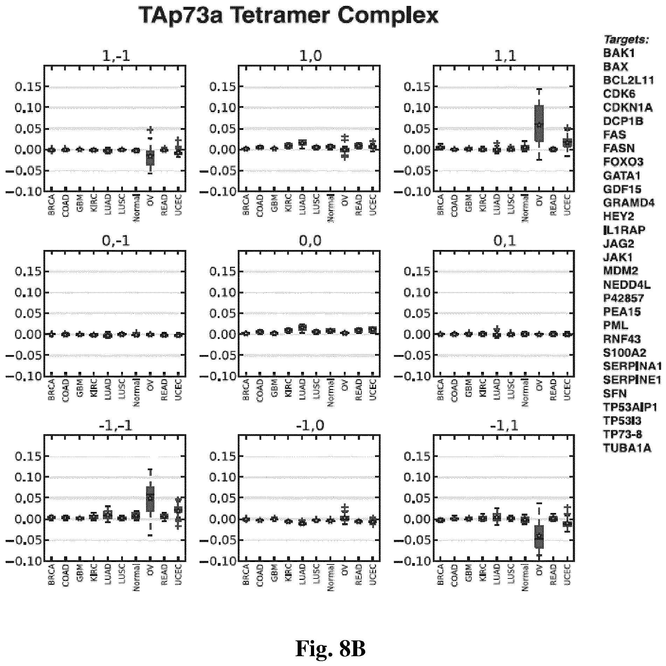

**Fig.7** shows tissue-differential link usage in the most significant by coloring each interaction by its correlation score in a tissue and setting its saturation proportional to its significance. The strongest differential g-scores were seen for links regulated by key cancer genes and complexes, including TP53, MYC/MAX, HIF1A/ARNT, TAp73a, E2F1 and PPARA-RXRA. Of particular interest are the links regulated by PPARA-RXRA primarily different within GBM [brain and KIRC (kidney)] and the TAp73a regulatory links in OV (ovarian) and to a lesser degree in UCEC (uterine endometrioid). **Figs. 8A and 8B** show a plot of the WPMI signals grouped by tissue for the activating links from PPARA-RXRA and TAp73a, where significantly increased weights are found on the activating diagonal, indicating increased use of these links as activators in those tissues. As can be taken from Fig. 8A showing WPMI values for links with PPARA:RXRA as a parent node, there is a stronger activation signal in GBM and KIRC, while Fig. 8B shows WPMI values for links with TAp73a as a parent node, indicating activation in OV.

The signature of TAp73 activity potentially indicates a female reproductive or hormonal pattern of pathogenesis associated with p73 expression. TAp73 promotes the expression of cell cycle inhibitors and inducers of apoptosis, one of which is the tumor suppressor BAX, which acts as an inhibitor of the activity of the oncogene BCL2. BCL2 is known to be highly expressed in serous ovarian cancer, and the results here show that although TAp73 is highly expressed and is a strong promoter of BAX expression (and thus BCL2 inhibition), it is nonetheless ineffective in retarding tumorigenesis, suggesting that small molecule inhibition of BCL2 may be equally ineffective. Not surprisingly, single-agent treatments of ovarian cancer with small molecule inhibitors of BCL2, despite high BCL2 expression in serous ovarian cancer, have not succeeded to date, suggesting a downstream blockade or attenuation of TAp73-mediated activity in this type of cancer. It is important to note that almost all of the serous ovarian samples here bore mutations in p53, perhaps suggestive of an upstream shunting of tumorigenesis as well that perhaps overcomes TAp73 over-expression or increased activity. Other groups have additionally shown the importance of PPARA-RXRA activity in both GBM and KIRC and their sensitivity to fenofibrate, a PPARA agonist. The tissue-specific signals identified through this analysis appear to reiterate recent biological discoveries that appear to be unique when examined in the context of the current TCGA dataset.

The most significant links learned across the entire TCGA cohort (see Table 1) are a number of known cancer genes including the forkhead box transcription factor A1, p53 and estrogen receptor alpha. To perform a gene set enrichment with DAVID on the genes involved in the 50 interactions with the highest G-scores, the inventors replaced families and complexes with their component genes. This produced 112 unique genes that were recognized by DAVID from the top 50 links. These genes were found to be significantly enriched ($P<1e^{-7}$) for a number of relevant KEGG terms including 'pathways in cancer', 'apoptosis', 'Jak-STAT signaling pathway' and 'MAPK signaling pathway' as well as a number of different cancer type-specific terms. The inventors then compared this result with what could be found by only looking at gene expression correlation of the genes that are linked in the pathway. The inventors needed to take the top 200 gene expression pairs by Pearson correlation from the flattened pathway to get a set of unique genes of comparable size (N=119) to the set produced by inventive algorithm. Although both gene sets produced similar enrichments for Gene Ontology terms for biological processes (GOTERM_BP_FAT), far fewer KEGG terms were found by using gene expression correlation than by using the learned links (20 versus 46 at FDR<0.05) and the FDR. The KEGG terms that overlapped between the two sets had a lower FDR in the determined set. To ensure that the flattening of families and complexes in the pathway was not biasing these results, the inventors repeated this analysis for non-family, non-complex links in the pathway only and found similar results (20 KEGG terms found for learned links versus 3 for expression correlation at FDR<0.05).

**Table 1.** Regulatory links with the highest g test score across the entire TCGA cohort

| Parent | Child | g score | Direction |
|---|---|---|---|
| FOXA1 | SFTPA (family):txreg | 3247.197 | ↑ |
| HNF1A | HNF4A (family):txreg | 3208.440 | ↑ |
| GATA1 | alpha-globin (family):txreg | 3065.885 | ↑ |
| ONECUT1 | HNF1B (family):txreg | 3008.945 | ↑ |
| p53 tetramer (complex) | MDM2:txreg[a] | 2931.148 | ↑ |
| KLF4 | Preproghrelin (family) :txreg | 2914.620 | ↑ |
| PDX1 | NR5A2 (family):txreg | 2872.275 | ↑ |
| p53 tetramer (complex) | SFN:txreg[a] | 2811.958 | ↑ |
| ER alpha homodimer (complex) | alpha tubulin (family):txreg | 2781.369 | ↑ |
| FOXM1 | CENPA:txreg | 2739.028 | ↑ |

*P*-values for all link are less than 1e-323.
[a]Intermediate node.

The inventors also compared the strength of links between subtypes of breast cancer to get some insight into the regulatory differences between the sub-types (see Table 2). This comparison as well as other comparisons between tissues never found links that completely switched direction from activation to inhibition. Instead, the inventors often observed that links turned off or on (e.g. changed from a strong activator to neutral). Because the direction rarely changes, the inventors found it informative to simply look at the differences between the G-score significance of links. The inventors used the rank difference of the G-scores to compare between groups so as to adjust for the G-score's dependence on sample size. Many of the links with the highest rank differences had the same parents. For that reason Table 2 shows the links with the highest rank difference on a per parent basis. In 9 of the top 10 links that were stronger in Basal tumors, HIF1A was the parent, and the top four links stronger in Luminal A tumors had CEBPB as a parent.

**Table 2.** Regulatory links with adjusted *P*<0.05 in either Basal (*N*=92) or Luminal A (*N*=218) breast cancer tumors, and the highest rank differences in G-scores per parent

| Parent | Child | *P*-value Basal | *P*-value Luminal | Rank difference | Direction |
|---|---|---|---|---|---|
| HIF1A/ARNT (complex) | HK1 | 1.61e-3 | 0.834 | 7826 | ↑ |
| E2F3/DP,TFE3 (complex) | RRM1 | 9.20e-3 | 0.854 | 7632 | ↑ |
| MYB | PPP3CA | 3.09e-2 | 0.493 | 5203 | ↑ |
| E2F1(DP (complex) | WASF1 | 3.48e-2 | 0.459 | 4924 | ↑ |
| E2F1/DP/PCAF (complex) | TP73 | 6.59e-3 | 0.343 | 4225 | ↑ |
| CEBPB | HSP90B1 | 0.879 | 9.65e-3 | 6275 | ↑ |
| JUN | AChR (family) | 0.833 | 0.0256 | 4742 | ↑ |
| SP1 | CDKN2C | 0.771 | 5.94e-4 | 4700 | Not significant |

(continued)

| Parent | Child | *P*-value Basal | *P*-value Luminal | Rank difference | Direction |
|---|---|---|---|---|---|
| DNA damage (abstract) | SERPINB5 | 0.808 | 0.0300 | 4264 | ↑ |
| LEF1/beta catenin/PITX2 (complex) | LEF1 | 0.775 | 9.18e-3 | 4250 | ↑ |

*Note*: Adjusted P of all rank differences in this table was < 4.8e-4. All edges were annotated as transcriptional activators. Full table is Supplementary Material.

To identify clinically relevant activities and link strengths, the inventors examined the estrogen receptor-positive (ER+) breast cancer patients and performed a regularized Cox regression of TCGA survival data on both link g-scores and IPLs to identify the optimal number of features to best split the cohort. At the minimum lambda, the coxnet model contained nine features that best split the ER+breast cancer patients (see Table 3). Four of the nine features were link g-scores, which illustrates the independent utility of these scores as potential prognostic markers.

**Table 3.** Pathway features (edges and nodes) associated with survival in ER + breast cancer patients

| Feature | Cox hazard coefficient |
|---|---|
| GLI2A → GLI1 | 0.08484 |
| HIF1A/ARNT (complex) → CP | 0.07835 |
| MYB → CEBPB | 0.00462 |
| E2F1/DP (complex) → SIRTI | -0.00072 |
| p300/CBP (complex) | -0.00204 |
| SDC3 | -0.04840 |
| p300/CBP/RELA/p50 (complex) | -0.11126 |
| TAp73a (tetramer) (complex) | -0.11301 |
| TCF1E/beta catenin (complex) | -0.16129 |

*Note*: Edges arc identified by →, and all edges found are annotated as transcriptional activators in the pathway.

CEBPB and HIF1A/ARNT appeared in both Tables 2 and 3. CEBPB is a transcription factor that has been associated with tumor progression, poor prognosis and ER negative status. Furthermore, over expression of HSP90B1, a heat shock protein regulated by CEBPB and found in Table 2, has been associated with distant metastases and decreased overall survival in breast cancer patients with otherwise good prognoses. HSP90B 1 has undergone clinical trials as an immunotherapy for melanoma under the name vitespen. HIF1A/ARNT overexpression is clinically relevant in ER- and PR- breast cancer, where splice variants have been associated with reduced metastasis-free survival. Because basal tumors are generally ER-, and Luminal A tumors are generally ER+, the differential link strength could be due to increased occurrence of the splice variant in the basal tumors. The top two links by G-score rank difference between basal and luminal are HIF1A/ARNT activating HK1 and HK2 (hexokinases), HK2 is involved in glucose metabolism and apoptosis, and has been associated with brain metastases from breast cancers as well as poor survival post craniotomy. These findings indicate the feasibility to find links that are relevant both by contrasting between tumor subtypes and by searching for links within a sub-type that are predictive of a clinical variable.

Based on the above, it should be appreciated that contemplated systems and methods allow for a combination of multiple -omics data to learn the strength and sign of regulatory interactions curated from the literature. The assumption of conditional independence enables a reduction in model complexity and allows efficient estimation of regulatory parameters using existing datasets. Moreover, the inventors also demonstrated that the independence assumption is valid for the vast majority of cellular regulatory programs. In addition, where the independence assumption does not hold, it is contemplated that independent factors could be replaced with more complex factors that properly model a co-dependent regulatory program. When these learned parameters are applied, biological insight can be gained from simply looking at the strongest links across a cohort of samples or by looking at how interactions change between phenotypes of interest.

It should also be appreciated that although cancer sub-types use different interactions, an interaction generally has a consistent sign whenever it is used in a particular tumor. Still further, the concordance of the learned interaction sign and the interaction sign in databases, despite the various ways that interaction sign is annotated in the BioPAX language across pathway databases, indicates that pathway databases have already successfully and faithfully cataloged of thousands of wetlab experiments in the literature.

In addition, it should be appreciated that the independence of co-regulators provides computational benefits for model inference and parameter learning, and also aids in model interpretation. The factorability of regulation models corresponds to log-linearity. However, a great number of regulators in the model are complexes, and the complex formation factor is a non-linear noisy-MAX function. Thus, regulation nonlinearity can still be encoded in the factor graph by representing physical complexes. This lends plausibility to a physical interpretation of most regulation links in the pathway: competitive binding of independent regulators should combine linearly, as long as the truly independent physical entities have been captured as complexes. If this physical interpretation is true, then there should be a correspondence between relative strengths of measured physical binding constants and determined interaction scores. In cases where the independence assumption does not hold, it is likely that there is a latent co-factor, which could be modeled by replacing $P(Y|X_1)P(Y|X_2)$ with a factor such as $P(Y|X_1,X_2)$.

Since contemplated methods and systems are capable of differentiating interaction correlations between tissue sub-types, the inventors also contemplate a method of classifying an omic dataset representative of a tissue (e.g., obtained from a tumor biopsy) as belonging to a sub-type specific tissue (e.g., as belonging to a treatment resistant tumor with respect to a particular drug). Similar to the methods discussed above, contemplated methods will first obtain via an omic input interface the omic dataset representative of the tissue, and then derive, for the omic dataset, a set of interaction correlations among a plurality of regulatory parameters of a regulatory node in a pathway model. The thusly derived set of interaction correlations is then matched to an *a priori* known set of interaction correlations that is associated with a known sub-type specific tissue, and where desired, the match is then used for classification of the omic dataset (e.g., to be representative of the known sub-type specific tissue, and with that to classify the tissue as belonging to the sub-type). Therefore, it should be appreciated that contemplated systems and methods will allow characterization of a tissue in terms of a sub-type merely based on one or more interaction correlation signatures. Among other contemplated tissue sub-types, especially advantageous sub-types include drug-resistant tissue, metastatic tissue, drug-treated tissue, or a clonal variant of a tissue.

Moreover, as contemplated systems and methods allow identification of signal flow through a signaling pathway and/or pathway network, it should be appreciated that contemplated systems and methods will also be useful to identify a druggable target in a pathway model. Such identification will typically include a steps of (a) obtaining, via an omic input interface, an omic dataset representative of a tissue, (b) deriving, for the omic dataset, a set of interaction correlations among the plurality of regulatory parameters of the regulatory node in the pathway model, and (c) identifying a drug as affecting the activity of the path where the drug is predicted to interfere with the interaction correlations. Most typically, the regulatory node affects at least one of transcription, translation, and post-translational modification of a protein, and the drug is a commercially available drug and has a known mode of action.

Thus, as specific interaction correlations among regulatory parameters of a pathway are known, target pathway in a pathway model can now be readily identified using an omic dataset representative of a tissue, and a derivation, for the omic dataset, of a set of interaction correlations among the regulatory parameters of a regulatory node in a pathway model. Where a drug has a known effect on the interaction correlation, the drug can then be used for targeting the target pathway. For example, the known effect of a drug may be an inhibitory effect on a kinase, an inhibitory effect on a receptor, and an inhibitory effect on transcription. Therefore, and among other suitable target pathways, especially contemplated target pathways include a calcium/calmodulin regulated pathway, a cytokine pathway, a chemokine pathway, a growth factor regulated pathway, a hormone regulated pathway, MAP kinase regulated pathway, a phosphatase regulated pathway, and a Ras regulated pathway. Depending on the outcome of the pathway analysis, treatment advice may then be based on the identified pathway.

Moreover, it should be appreciated that treatment need not be actually performed in a patient, but may be simulated once one or more specific interaction correlations among regulatory parameters of a pathway are known. Such simulation may be used to predict treatment outcome or identification of multiple drugs to effectively low of signals through the pathways. Thus, contemplated methods will also include a method of *in silico* simulating a treatment effect of a drug that includes a step of obtaining a pathway model having a plurality of pathway elements in which at least two of the elements are coupled to each other via a path having a regulatory node that controls activity along the path as a function of a plurality of regulatory parameters. Contemplated methods will further include a step of identifying a drug that is known to affect at least one regulatory parameter, and another step of altering *in silico,* via an omic processing module and based on the known effect of the drug, at least one of the regulatory node, the activity, and at least of the regulatory parameters in the pathway model, and yet another step of determining a secondary effect of the alteration in the pathway model. Most typically, the secondary effect is in another regulatory node, another activity, and another regulatory parameter in the pathway model.

It should be apparent to those skilled in the art that many more modifications besides those already described are possible without departing from the inventive concepts herein. Moreover, in interpreting both the specification and the claims, all terms should be interpreted in the broadest possible manner consistent with the context. In particular, the terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Where the specification claims refers to at least one of something selected from the group consisting of A, B, C .... and N, the text should be interpreted as requiring only one element from the group, not A plus N, or B plus N, etc.

**Claims**

1. A computer-implemented method of classifying a tissue as belonging to a sub-type specific tissue, comprising:

   (a) obtaining, via an omic input interface module (120), at least one omic dataset (135) representative of the tissue;
   (b) accessing, via an omic processing module (170), a biological pathway model (150) having a plurality of pathway elements comprising at least one of a DNA sequence, an RNA sequence, a protein, and a protein function, in which at least two of the elements are coupled to each other via a path having a regulatory node that controls activity along the path as a function of a plurality of regulatory parameters;

   (i) when the pathway element comprises a DNA sequence, the at least one of the plurality of regulatory parameters is selected from the group consisting of a transcription factor, a transcription activator, a RNA polymerase subunit, a cis-regulatory element, a trans-regulatory element, an acetylated histone, a methylated histone, and a repressor,
   (ii) when the pathway element comprises a RNA sequence, the at least one of the plurality of regulatory parameters is selected from the group consisting of an initiation factor, a translation factor, a RNA binding protein, a ribosomal protein, an siRNA, and a polyA binding protein, and
   (iii) when the pathway element comprises a protein, the at least one of the plurality of regulatory parameters is a phosphorylation, an acylation, a proteolytic cleavage, and association with at least a second protein;

   (c) inferring, via the omic processing module (170), based on the at least one omic dataset (135) and the pathway model (150), a set of interaction correlations among the plurality of regulatory parameters, wherein conditional probabilities of individual links are learned and a Naive Bayes assumption is used to calculate the probability of a child node Y given the parents $X_1, ... X_n$, wherein the probability F is calculated on the basis of the expression:

$$F(Y|X_1, \ldots, X_N) = \frac{1}{Z} P(Y) \prod_i P(X_i|Y)$$

   wherein Z is a normalizing constant that corresponds to $P(X_1, ... X_n)$, the pathway model being a probabilistic model configured to use factor graphs using an independent regulation model;
   (d) updating the pathway model (150) based on the learned interaction correlations,
   wherein the omic input interface is a computing interface configured to receive one or more of omic datasets, and wherein the omic processing module is a portion of a computing device,
   wherein a G-test determines statistical significance of the dependence between parents given a child distribution, and
   wherein a Pearson correlation or weighted pointwise mutual information (WPMI) determines the sign of interaction for the regulatory parameters,
   (e) matching the derived set of interaction correlations to an a priori known set of interaction correlations that is associated with a known sub-type specific tissue; and
   (f) using the match to classify that omic dataset representative of the tissue belongs to the known sub-type specific tissue, wherein the sub-type includes drug-resistant tissue, metastatic tissue, drug-treated tissue or a clonal variant of a tissue.

2. The computer-implemented method of claim 1 wherein the omic datasets (135) comprise whole genome data, partial genome data, or differential sequence objects, and wherein the omic datasets (135) are obtained from a genomic database (130), a BAM server (130), or a sequencing device (130).

3. Apparatus configured to carry out the method of claim 1 or claim 2.

**Patentansprüche**

1. Computerimplementiertes Verfahren zum Klassifizieren eines Gewebes als zu einem Untertyp-spezifischen Gewebe gehörend, umfassend:

   (a) Erlangen, über ein omisches Eingangsschnittstellenmodul (120), von mindestens einem Datensatz (135), der für das Gewebe repräsentativ ist;

   (b) Zugreifen, über ein omisches Verarbeitungsmodul (170), auf ein biologisches Pfadmodell (150) mit einer Vielzahl von Pfadelementen, die mindestens eines von einer DNA-Sequenz, einer RNA-Sequenz, einem Protein und einer Proteinfunktion umfassen, wobei mindestens zwei der Elemente über einen Pfad mit einem regulatorischen Knoten miteinander gekoppelt sind, der eine Aktivität entlang des Pfads abhängig von einer Vielzahl von regulatorischen Parametern steuert;

   (i) wenn das Pfadelement eine DNA-Sequenz umfasst, der mindestens eine der mehreren regulatorischen Parameter aus der Gruppe ausgewählt ist, die aus einem Transkriptionsfaktor, einem Transkriptionsaktivator, einer RNA-Polymerase-Untereinheit, einem *cis*-regulierenden Element, einem *trans*-regulatorischen Element, einem acetylierten Histon, einem methylierten Histon und einem Repressor besteht,

   (ii) wenn das Pfadelement eine RNA-Sequenz umfasst, der mindestens eine der mehreren regulatorischen Parameter aus der Gruppe ausgewählt ist, die aus einem Initiationsfaktor, einem Translationsfaktor, einem RNA-bindenden Protein, einem ribosomalen Protein, einer siRNA und einem PolyA-bindenden Protein besteht, und

   (iii) wenn das Pfadelement ein Protein umfasst, der mindestens eine der mehreren regulatorischen Parameter eine Phosphorylierung, eine Acylierung, eine proteolytische Spaltung und eine Assoziierung mit mindestens einem zweiten Protein ist;

   (c) Stören, über das omische Verarbeitungsmodul (170), beruhend auf dem mindestens einen omischen Datensatz (135) und dem Pfadmodell (150), eines Satzes von Wechselwirkungskorrelationen zwischen der Vielzahl von regulatorischen Parametern, wobei konditionale Wahrscheinlichkeiten einzelner Verbindungen gelernt werden und eine Naive Bayes-Annahme verwendet wird, um die Wahrscheinlichkeit eines Kindknotens Y zu berechnen, der den Eltern $X_1 .... X_n$ gegeben ist, wobei die Wahrscheinlichkeit F auf Grundlage des folgenden Ausdrucks berechnet wird:

   $$F(Y|X_1, \ldots, X_N) = \frac{1}{Z} P(Y) \prod_i P(X_i|Y)$$

   wobei Z eine Normierungskonstante ist, die $P(X_1, ... X_n)$ entspricht, wobei das Pfadmodell ein probabilistisches Modell ist, das für die Verwendung von Faktorkurven unter Verwendung eines unabhängigen Regulierungsmodells konfiguriert ist;

   (d) Aktualisieren des Pfadmodells (150) beruhend auf den erlernten Interaktionskorrelationen, wobei die omische Eingabeschnittstelle eine Rechenschnittstelle ist, die konfiguriert ist, um einen oder mehrere omische Datensätze zu empfangen, und wobei das omische Verarbeitungsmodul ein Teil einer Rechenvorrichtung ist, wobei ein G-Test eine statistische Signifikanz der Abhängigkeit zwischen Eltern bei einer Kinderverteilung bestimmt, und wobei eine Pearson-Korrelation oder gewichtete punktweise gegenseitige Information (weighted pointwise mutual information, WPMI) das Vorzeichen der Interaktion für die regulatorischen Parameter bestimmt,

   (e) Anpassen des abgeleiteten Satzes von Interaktionskorrelationen an einen *a priori* bekannten Satz von Interaktionskorrelationen, der mit einem bekannten Untertyp-spezifischen Gewebe assoziiert ist; und

   (f) Verwenden der Übereinstimmung, um zu klassifizieren, dass der für das Gewebe repräsentative omische Datensatz zu dem bekannten Untertyp-spezifischen Gewebe gehört, wobei der Untertyp medikamentenresistentes Gewebe, metastatisches Gewebe, medikamentenbehandeltes Gewebe oder eine klonale Variante eines Gewebes enthält.

2. Computerimplementiertes Verfahren nach Anspruch 1, wobei die omischen Datensätze (135) ganze Genomdaten, Teilgenomdaten oder differentielle Sequenzobjekte umfassen, und wobei die omischen Datensätze (135) aus einer genomischen Datenbank (130), einem BAM-Server (130) oder einer Sequenzierungsvorrichtung (130) erlangt werden.

3. Gerät, das konfiguriert ist, um das Verfahren nach Anspruch 1 oder Anspruch 2 durchzuführen.

**Revendications**

1. Procédé mis en oeuvre par ordinateur de classification d'un tissu comme appartenant à un tissu spécifique d'un sous-type, comprenant :

(a) l'obtention, par un module d'interface d'entrée omique (120), d'au moins un ensemble de données omiques (135) représentatif du tissu ;
(b) l'accès, par un module de traitement omique (170), à un modèle de voies biologiques (150) ayant une pluralité d'éléments de voies comprenant au moins une parmi une séquence d'ADN, une séquence d'ARN, une protéine et une fonction des protéines, dans lequel au moins deux des éléments sont couplés l'un à l'autre par une voie ayant un noeud régulateur qui commande une activité le long de la voie comme une fonction d'une pluralité de paramètres régulateurs ;

(i) quand l'élément de voie comprend une séquence d'ADN, le au moins un de la pluralité de paramètres régulateurs est sélectionné parmi le groupe constitué d'un facteur de transcription, d'un activateur de transcription, d'une sous-unité de polymérase d'ARN, d'un élément cis-régulateur, d'un élément trans-régulateur, d'une histone acétylée, d'une histone méthylée et d'un répresseur,
(ii) quand l'élément de voie comprend une séquence d'ARN, le au moins un de la pluralité de paramètres régulateurs est sélectionné parmi le groupe constitué d'un facteur d'initiation, d'un activateur de traduction, d'une protéine de liaison à l'ARN, d'une protéine ribosomale, d'un siARN et d'une protéine de liaison polyA, et
(iii) quand l'élément de voie comprend une protéine, le au moins un d'une pluralité de paramètres régulateurs est une phosphorylation, une acylation, un clivage protéolytique et une association avec au moins une seconde protéine ;

(c) l'inférence, par le module de traitement omique (170), basée sur le au moins un ensemble de données omiques (135) et le modèle de voie (150), d'un ensemble de corrélations d'interactions parmi la pluralité de paramètres régulateurs, dans lequel des probabilités conditionnelles de liens individuels sont assimilées et une hypothèse bayésienne naïve est utilisée pour calculer la probabilité d'un noeud enfant Y étant donné les parents $X_1, ... X_n$, dans lequel la probabilité F est calculée sur la base de l'expression :

$$F(Y|X_1, \ldots, X_N) = \frac{1}{Z} P(Y) \prod_i P(X_i | Y)$$

dans lequel Z est une constante de normalisation qui correspond à $P(X_1, ... X_n)$, le modèle de voie étant un modèle probabiliste configuré pour utiliser des graphes de facteurs utilisant un modèle de régulation indépendant ;
(d) la mise à jour du modèle de voie (150) basé sur les corrélations d'interaction assimilées, dans lequel l'interface d'entrée omique est une interface informatique configurée pour recevoir un ou plusieurs ensembles de données omiques, et
dans lequel le module de traitement omique est une partie d'un dispositif informatique,
dans lequel un test G détermine une signification statistique de la dépendance entre des parents donnés dans une distribution des enfants, et
dans lequel une corrélation de Pearson ou des informations mutuelles instantanées pondérées (WPMI) détermine le signe d'interaction des paramètres régulateurs,
(e) l'établissement d'une correspondance d'un ensemble dérivé de corrélations d'interactions avec un ensemble connu a priori de corrélations d'interactions qui est associé avec un tissu spécifique d'un sous-type connu ; et
(f) l'utilisation de la correspondance pour classer qu'un ensemble de données omiques représentatif du tissu appartient au tissu spécifique d'un sous-type connu, dans lequel le sous-type comprend un tissu résistant aux médicaments, un tissu métastatique, un tissu traité par médicaments ou une variante clonale d'un tissu.

2. Procédé mis en oeuvre par ordinateur selon la revendication 1, dans lequel les ensembles de données omiques (135) comprennent des données de génome entier, des données de génome partiel ou des objets de séquence différentielle, et dans lequel les ensembles de données omiques (135) sont obtenus depuis une base de données génomiques (130), un serveur BAM (130) ou un dispositif de séquençage (130).

3. Appareil configuré pour exécuter le procédé selon la revendication 1 ou la revendication 2.

**Fig. 1**

**Fig. 2A**

Co-dependent Regulation Model     Independent Regulation Model

**Fig. 2B**

**Fig. 3**

**Fig. 4**

**Fig. 5**

**A**

Kaplan–Meier curve

log–rank p: 5.20e–02
cox p: 5.36e–02
hopach_cosangle_1000 (n = 60 )
hopach_cosangle_2000 (n = 127 )
hopach_cosangle_3000 (n = 83 )
hopach_cosangle_4000 (n = 125 )

Original Regulation Model

**Fig. 6A**

**B**

Kaplan–Meier curve

log–rank p: 7.81e–02
cox p: 7.95e–02
hopach_cosangle_10 (n = 70 )
hopach_cosangle_20 (n = 113 )
hopach_cosangle_60 (n = 93 )
hopach_cosangle_70 (n = 119 )

Co-dependent Regulation Model

**Fig. 6B**

Independent Regulation Model

Fig. 6C

Fig. 7

Fig. 8A

Fig. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2011139345 A

- WO 2013062505 A